# EUROPEAN PATENT SPECIFICATION

(11) **EP 2 293 821 B1**
(45) Date of publication and mention of the grant of the patent: **28.08.2013**
(21) Application number: 08873683.0
(22) Date of filing: 09.06.2008
(51) Int. Cl.: A61L 2/26, A61L 2/07, B65B 55/18

(54) **STERILE PACKING AND STERILIZATION METHOD USING THIS PACKING**
STERILISIERTE VERPACKUNG UND VERFAHREN ZU IHRER VERWENDUNG
EMBALLAGE STÉRILE ET PROCÉDÉ DE STÉRILISATION UTILISANT LEDIT EMBALLAGE

(43) Date of publication of application: 16.03.2011
(73) Proprietor: Becton Dickinson France, 38800 Le Pont de Claix (FR)
(72) Inventor: MERMET, Emeric, 38000 Grenoble (FR)
(74) Representative: Jeannet, Olivier
(86) International application number: PCT/IB2008/002608
(87) International publication number: WO 2009/150487

(56) References cited:
- EP-A- 1 520 795
- WO-A-2004/039419
- DE-A1-102006 027 304
- US-A- 4 352 429

## Description

The present invention concerns a sterile packing and a sterilization method using this packing.

Some activities involve transporting sterile parts or components in sterile packing. This is the case in particular for the component parts of syringes, which must be transported between the production site and an assembly site, to form the syringe, and fill the syringe bodies.

A known sterilization method used for syringe parts consists of placing these parts in packings made of a flexible and airtight material, then exposing these packings thus filled to gamma rays. This method has the drawback, for the syringe manufacturer, of having to pack the non-sterilized parts in non-sterile packings, then to transmit these packings to a service provider specialized in this type of sterilization, which, after sterilization, transmits these packings to the purchaser of the parts, for assembly and/or filling of the syringes. The use of a specialized subcontractor of this type constitutes a notable constraint for the syringe manufacturer.

Another known sterilization method in such an application uses water vapor to sterilize the parts and their packing. This sterilization method is preferred to the radiation sterilization method because it is well-received by the pharmaceutical industry using the syringes, or even required by some users, or is also made obligatory by the nature or material of the packed parts or components. There are not, however, packings making it possible to ensure the perfect performance of sterilization during the sterilization method and, after transport, the perfect preservation of the integrity of the packing all the way to the end user.

The documents EP 1 520 795, US 4 352 429 and WO 2004/039419 disclose various packings according to the prior art.

The present invention aims to resolve the abovementioned drawbacks.

Its objective is therefore to provide a packing making it possible to ensure the perfect performance of a sterilization of one or several objects to be sterilized, in particular by water vapor, to ensure the perfect preservation of sterility during transport and storage of the packing, and to immediately detect any loss of integrity of the packing and therefore any loss of the sterility thereof. The aim of the invention is also to provide a sterilization method using this packing, making it possible to sterilize said objects while ensuring the perfect performance of the sterilization thereof.

The term "object" will be used below generically to generally designate one or several parts or one or several components to be packed; this term must be understood in the broadest sense, covering all types of parts, products or components, and in particular the component parts of syringes.

To achieve the aforementioned objective, the packing according to the invention comprises:
- a first part, made of flexible and airtight material, defining a first portion and a second portion contiguous to the first portion, said first portion being separated from the second portion by an area suitable to be folded;
- at least one second part, made of a material porous to the sterilizing fluid, forming a pocket for the reception of the at least one object to be sterilized, said second part being connected to said first portion of said first part and being dimensioned so that its edges are, when said second part is connected to said first portion, set back of the edges of said first portion so as to define side assembly areas on said first portion; and
- airtight connection means for the connection of said second portion to said first portion in said side assembly areas.

The sterilization and packing method according to the invention comprises the steps of:
- placing one or several objects to be sterilized in the pocket formed by said at least one second part;
- sterilizing the unit by means of the sterilization fluid;
- folding up said second portion on said first portion, and
- carrying out the assembly of this second portion onto this first portion in the side assembly areas with said connection means.

The invention thus consists of using a packing comprising a pocket in a porous material, permeable to the sterilization fluid, to contain one or several objects to be sterilized, which is connected to said first portion of a part made of an airtight material; after sterilization, said second portion is folded up onto said pocket so as to cover all of this pocket and is then sealably connected to said side assembly areas of said first portion, thereby isolating the pocket relative to the outside.

The porosity of the pocket allows a sufficient diffusion of sterilization fluid inside this pocket and around all of the objects contained in this pocket; the airtight connection of said second portion to said first portion makes it possible to perfectly protect these objects with regard to the outside environment and to preserve the integrity of the sterilization done.

The packing and the method according to the invention therefore have the determining advantages of allowing effective sterilization of objects by the sterilization fluid, perfectly preserving the integrity of the packed objects, and making it possible to immediately show any loss of integrity, and therefore of sterility, of the packing.

The material of said second portion comprises pores whereof the size can go from 2 to 15 microns and a Log Reduction Value (as defined in the ASTM F-1608 standard) greater than or equal to 3. This can be a film marketed by the company Du Pont De Nemours under the TYVEK® brand, references 1073B, 2FS or 1059B, or the complex marketed by the company WIPAK under the brand WIPAK®, with references Paper 80B or Paper 120B.

The packing can comprise a third part, made of a flexible and airtight material, dimensioned to be able to contain said first part when folded on said second part, this third part being closed on said first part when folded on said second part and being airtightly sealed with a vacuum created between said third part and said first part.

This third part strengthens the resistance of the packing to the stresses this packing may undergo during its transport and handling, and the air vacuum created between it and said first part constitutes, at the time of opening of the packing, a demonstration of the preservation of the integrity of this packing, and therefore the preservation of the perfect sterility thereof.

As a result, in this case the method comprises the steps consisting of:
- using said third part;
- folding said third part on said first part when this first part is folded on said second part;
- creating a vacuum between said third part and said first part, and
- tightly sealing said third part while maintaining the vacuum between said third part and said first part.

The assembly formed by said first part when it is folded on said second part can be independent of said third part; this assembly can also be connected to this third part, in particular by connecting said side assembly areas to said third part.

Said second part(s) can form said pocket by themselves, for example being folded and being connected to each other on their side edges; said second part(s) can also be flat and be connected to said first part at their perimeter, so as to form said pocket and the opening of this pocket.

Preferably, said first part is made of a thermoweldable material and said sealing connection means include this material itself, the assembly of said first portion and said second portion of this first part being carried out by thermowelding.

Also preferably, said at least one second part is made of thermoweldable material and its connection to said first part is done by thermowelding.

The packing according to the invention can thus be produced particularly simply.

Said first part and said second part could have any shape of a nature to pack the concerned objects according to the invention. According to one simple embodiment of the packing according to the invention, said first part has a rectangular shape and said first and second portions of this part are defined by the two portions of said first part extending on both sides of a transverse median axis, this transverse median axis corresponding to said area suitable to be folded.

Preferably, said first part is made of several superimposed layers of flexible and airtight material.

This plurality of layers minimizes the risk of a loss of integrity of the packing if a hole appears in one layer. Moreover, the risk of having aligned holes decreases as the number of layers increases.

The invention will be well understood, and other characteristics and advantages thereof will appear, in reference to the appended diagrammatic drawing, illustrating, as non-limiting examples, two possible embodiments of the sterile packing it concerns.
Figure 1 is a flat view of two material parts making up this packing, according to a first embodiment, before filling of the packing;
figure 2 is a side view of these parts;
figure 3 is a view of these parts similar to figure 2, after filling by the objects to be sterilized;
figure 4 is a view of these parts similar to figure 3, after sealing of one of these parts;
figure 5 is a top view of these parts, after said sealing;
figure 6 is a side view of the assembly shown in figures 4 and 5, after placement in an envelope formed by a third part;
figure 7 is a view of the packing similar to figure 6, after sealing of said envelope, and
figures 8 to 10 are views similar to figures 2, 4 and 7 of the packing, respectively, according to a second embodiment.

For simplification, the parts or elements of one embodiment which are found identically or similarly in the other embodiment will be identified using the same numerical references and will not be described again.

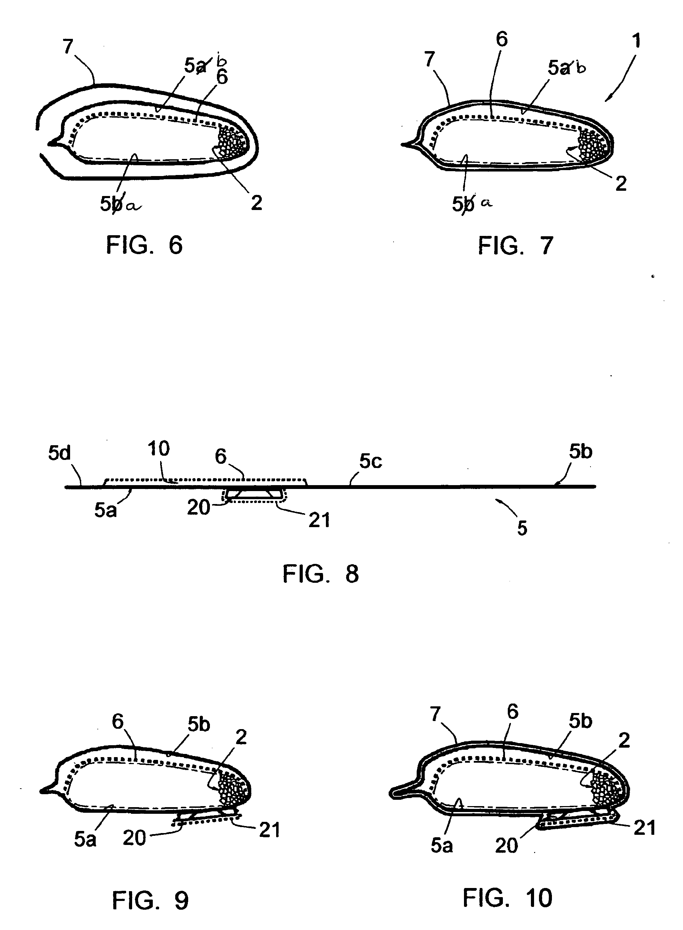

Figure 7 illustrates a packing 1 designed to contain objects 2, in particular component elements of syringes, and in particular syringe plungers. These objects 2 together fill a pocket comprised by the packing 1 but, out of a concern for clarity in the drawing, they have only been partially shown: the overall contour formed by these objects 2 is defined by a dashed line.

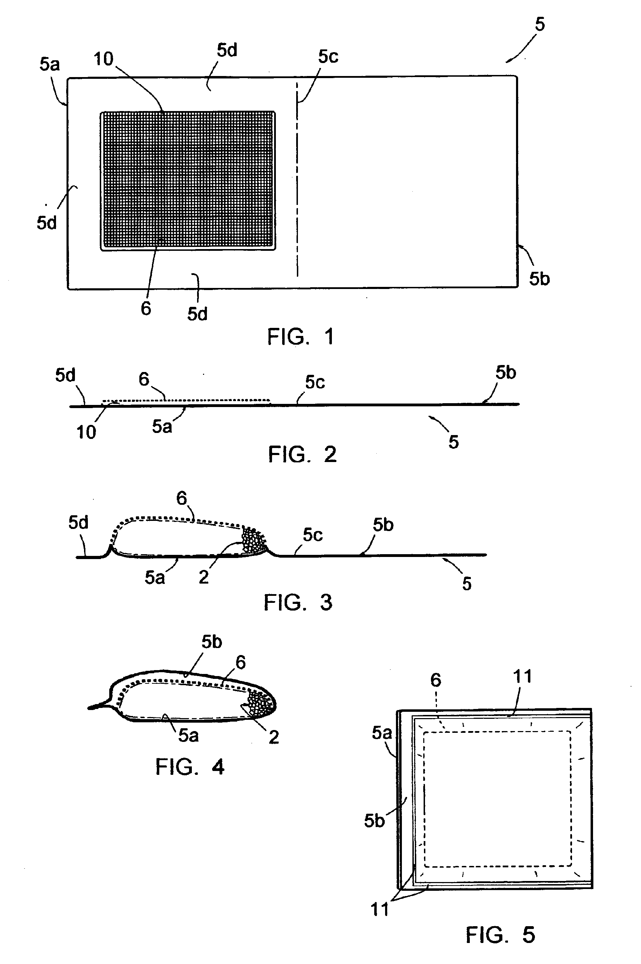

In reference to figures 1 to 7, it appears that the packing 1 comprises three material parts 5, 6 and 7.

The part 5 is made of a flexible and airtight material, in particular a thermoweldable synthetic material. It is rectangular and defines a first portion 5a and a second portion 5b contiguous to the first portion 5a, said first portion 5a being separated from the second portion 5b by a median area 5c able to be folded.

The part 6 is made of a material porous to a sterilization fluid such as water vapor and non-porous to microbic contamination. This material comprises pores whereof the size can go from 2 to 15 microns and a Log Reduction Value (as defined in the ASTM F-1608 standard) greater than or equal to 3. This can be a film marketed by the company Du Pont De Nemours under the TYVEK® brand, references 1073B, 2FS or 1059B, or a complex marketed by the company WIPAK under the WIPAK® brand, reference Paper 80B or Paper 120B.

This part 6 is rectangular in shape and has dimensions smaller than those of said first portion 5a; it is connected on three of its sides to this first portion 5a, while being positioned set back from the edges thereof other than the one connected to said median area 5c, so as to define three side assembly areas 5d on said first portion 5a. Through this connection on three of its sides to said first portion 5a, the part 6 forms a pocket 10 for receiving objects 2 to be sterilized. The connection of the part 6 to the part 5 can be done using any suitable means, in particular gluing or thermowelding.

After filling the pocket 10 with the objects 2 (cf. figure 3) and placing the assembly, in this state, in a sterilization chamber, the portion 5b of the part 5 is folded on the pocket 10 and on the portion 5a, then is heat-sealed to the side assembly areas 5d. The heat-sealed areas 11 extend continuously from one end of the folding area 5c to the other end, including the pocket 10, such that this pocket 10 and the objects 2 it contains are perfectly tightly isolated from the ambient air.

The assembly thus formed is then placed in an envelope formed by said material part 7 (cf. figure 6), this part 7 being made of a flexible and airtight material, in particular a thermoweldable synthetic material.

The envelope formed by this part 7 is then closed on all of said assembly (cf. figure 7), then is airtightly heat-sealed with a vacuum created between the part 7 and the part 5.

As appears from the preceding, the porosity of the part 6 allows a great diffusion of the sterilization fluid inside the pocket 10 and around all of the objects 2 contained therein, sufficient to ensure the sterilization of these objects 2; the airtight connection of said second portion 5b to said first portion 5a enables perfect protection of these objects 2 with regard to the outside environment and preservation of the integrity of the sterilization done.

The part 7 strengthens the resistance of the packing 1 to the stresses this packing may undergo during its transport and handling, and the vacuum created between it and the part 5 constitutes, when the packing 1 is opened, a demonstration of the preservation of the integrity of this packing, and therefore the preservation of the perfect sterility thereof.

Figures 8 to 10 illustrate a packing 1 identical to that which was just described, except that the part 5 comprises a ring 20 for connecting the packing 1 to a sterile chamber wherein the objects 2 are designed to be transferred. This ring 20 comprises a circular base defining an opening for pouring of the objects 2 and a removable door, which, at this stage of the use of the packing 1, closes this pouring opening. The ring 20 is, for example, of the type described in documents US 6,571,540 and US 6,817,143, and therefore will not be described in further detail.

As appears in figures 8 to 10, the ring 20 is covered by a membrane 21, which is porous to the sterilization fluid, this membrane 21 allowing sterilization of the ring 20 at the same time the sterilization of the objects 2 is done. This membrane can in particular be made of the same material as the part 6.

The invention thus provides a sterile packing and a sterilization method using this packing, having the determining advantages of allowing effective sterilization of objects by the sterilization fluid, perfectly preserving the integrity of the packed objects, and making it possible to immediately detect any loss of integrity, and therefore sterility, of the packing.

It must be specified that the embodiment of the invention described above was provided purely as an example. It goes without saying that the invention is not limited to this embodiment, but that it extends to all embodiments covered by the appended claims. Thus, the packing 1 can comprise several parts 6; said part(s) 6 can form said pocket by themselves, being folded and being connected to each other on the side edges then being connected to the part 5; a part 6 can be connected to the portion 5a of the part 5 on its four sides and comprise a slot, in particular median, forming the opening of the pocket 10; the part 5 can be made of several superimposed layers.

## Claims

1. - Sterile packing (1) for containing at least one object (2) to be sterilized by a sterilizing fluid, including:
- a first part (5) of flexible and airtight material, defining a first portion (5a) and a second portion (5b) contiguous to the first portion (5a), said first portion (5a) being separated from said second portion (5b) by an area (5c) suitable to be folded , and
- at least one second part (6), made of material porous to the sterilizing fluid, connected to the first portion (5a) of said first part (5);
**characterized in that**:
- said at least one second part (6) forms a pocket (10) for the reception of the at least one object (2) to be sterilized, said second part (6) being dimensioned so that its edges are set back of the edges of said first portion (5a) so as to define on said first portion (5a) side assembly areas (5d), said second portion (5b) being foldable over the pocket (10) and said side assembly areas (5d) and dimensioned so as to cover all of this pocket (10) and said side assembly areas (5d), and being sealably connected to said side assembly areas (5d) of said first portion (5a), thereby isolating the pocket (10) relative to the outside, and
- the packing (1) includes connection means (11) for the sealed connection of said second portion (5b) to said first portion (5a) in said side assembly areas (5d).

2. - Sterile packing (1) according to claim 1, **characterized in that** it includes a third part (7), made of a flexible and airtight material, dimensioned to be able to contain said first part (5) when folded on said second part (6), this third part (7) being closed on said first part (5) when folded on said second part (6) and being sealed with a vacuum created between said third part (7) and said first part (5).

3. - Sterile packing (1) according to claim 1 or claim 2, **characterized in that** said at least one second part (6) forms by itself said pocket (10).

4. - Sterile packing (1) according to claim 1 or claim 2, **characterized in that** said at least one second part (6) is flat and is connected to said first part (5) on the periphery thereof, so as to form said pocket (10) and the opening of this pocket (10).

5. - Sterile packing (1) according to one of claims 1 to 4, **characterized in that** said first part (5) is made of thermoweldable material and **in that** said connection means includes this material itself, the assembly of said the first portion (5a) and the second portion (5b) of this first part (5) being carried out by thermowelding.

6. - Sterile packing (1) according to one of claims 1 to 5, **characterized in that** said second part (6) is made of thermoweldable material and **in that** its connection to said first part (5) is carried out by thermowelding.

7. - Sterile packing (1) according to one of claims 1 to 6, **characterized in that** said first part (5) has a rectangular shape and **in that** said first and second portions (5a, 5b) of this first part (5) are defined by the two portions of said first part (5) extending on both sides of a transverse median axis, this transverse median axis corresponding to said area (5c) suitable to be folded.

8. - Sterile packing (1) according to one of claims 1 to 7, **characterized in that** said first part (5) is made of several superimposed layers of flexible and airtight material.

9. - Sterilization process, using a sterile packing (1) according to one of claims 1 to 8, **characterized in that** it includes the steps consisting:
- to place the at least one object (2) to be sterilized in the pocket (10) formed by said at least one second part (6);
- to sterilize this unit by means of the sterilization fluid;
- to fold up said second portion (5b) on said first portion (5a), and
- to carry out the assembly of this second portion (5b) onto this first portion (5a) in said side assembly areas (5d) with said connection means (11).

10. - Process according to claim 9, **characterized in that** it includes the steps consisting:
- to use said third part (7);
- to fold this third part (7) on said first part (5) when folded on said second part (6);
- to create a vacuum between said third part (7) and said first part (5), and
- to seal said third part (7) while maintaining the vacuum between said third part (7) and said first part (5).

11. - Process according to claim 10, **characterized in that** it includes the step consisting:
- to connect the unit formed by said first part (5) when folded on said second part (6) to said third part (7), in particular by connecting said side assembly areas (5d) to this third part (7).

## Patentansprüche

1. - Sterile Verpackung (1) zum Aufnehmen mindestens eines Objekts (2), das durch eine Sterilisierungsflüssigkeit zu sterilisieren ist, umfassend:
- einen ersten Teil (5) flexiblen und luftdichten Materials, der einen ersten Abschnitt (5a) und einen mit dem ersten Abschnitt (5a) zusammenhängenden zweiten Abschnitt (5b) aufweist, wobei der erste Abschnitt (5a) von dem zweiten Abschnitt (5b) durch eine Fläche (5c) getrennt ist, die geeignet ist, gefaltet zu werden, und
- mindestens einen zweiten Teil (6), der aus einem Material hergestellt ist, das durchlässig für die Sterilisierungsflüssigkeit ist, und der mit dem ersten Abschnitt (5a) des ersten Teils (5) verbunden ist;
**dadurch gekennzeichnet, dass**:
- der mindestens eine zweite Teil (6) eine Tasche (10) zur Aufnahme des mindestens einen Objekts (2), das zu sterilisieren ist, bildet, wobei der zweite Teil (6) so bemessen ist, dass seine Kanten gegenüber den Kanten des ersten Abschnitts (5a) zurückgesetzt sind, so dass auf dem ersten Abschnitt (5a) Seitenmontageflächen (5d) ausgebildet sind, wobei der zweite Abschnitt (5b) über die Tasche (10) und die Seitenmontageflächen (5d) faltbar ist und so bemessen ist, dass die gesamte Tasche (10) und die Seitenmontageflächen (5d) bedeckt werden, und dichtend mit den Seitenmontageflächen (5d) des ersten Abschnitts (5a) verbunden ist, wodurch die Tasche (10) in Bezug auf die Außenseite isoliert ist, und
- die Verpackung (1) Verbindungsmittel (11) für die abgedichtete Verbindung des zweiten Abschnitts (5b) mit dem ersten Abschnitt (5a) in den Seitenmontageflächen (5d) umfasst.

2. - Sterile Verpackung (1) nach Anspruch 1, **dadurch gekennzeichnet, dass** sie einen dritten Teil (7) umfasst, der aus einem flexiblen und luftdichten Material hergestellt und so bemessen ist, dass er fähig ist, den ersten Teil (5) aufzunehmen, wenn er um den zweiten Teil (6) gefaltet ist, wobei dieser dritte Teil (7) den ersten Teil (5) abschließt, wenn er um den zweiten Teil (6) gefaltet ist, und mit einem zwischen dem dritten Teil (7) und dem ersten Teil (5) erzeugten Vakuum abgedichtet ist.

3. - Sterile Verpackung (1) nach Anspruch 1 oder Anspruch 2, **dadurch gekennzeichnet, dass** der mindestens eine zweite Teil (6) an sich die Tasche (10) bildet.

4. - Sterile Verpackung (1) nach Anspruch 1 oder Anspruch 2, **dadurch gekennzeichnet, dass** der mindestens eine zweite Teil (6) flach und mit dem ersten Teil (5) an dessen Umfang verbunden ist, um die Tasche (10) und die Öffnung dieser Tasche (10) zu bilden.

5. - Sterile Verpackung (1) nach einem der Ansprüche 1 bis 4, **dadurch gekennzeichnet, dass** der erste Teil (5) aus einem thermoschweißbaren Material hergestellt ist und das Verbindungsmittel dieses Material selbst umfasst, wobei das Zusammenfügen des ersten Abschnitts (5a) und des zweiten Abschnitts (5b) dieses ersten Teils (5) durch Thermoschweißen erfolgt.

6. - Sterile Verpackung (1) nach einem der Ansprüche 1 bis 5, **dadurch gekennzeichnet, dass** der zweite Teil (6) aus einem thermoschweißbaren Material hergestellt ist und seine Verbindung mit dem ersten Teil (5) durch Thermoschweißen erfolgt.

7. - Sterile Verpackung (1) nach einem der Ansprüche 1 bis 6, **dadurch gekennzeichnet, dass** der erste Teil (5) eine rechteckige Form aufweist und die ersten und zweiten Abschnitte (5a, 5b) dieses ersten Teils (5) als zwei Abschnitte des ersten Teils (5) ausgebildet sind, die sich auf beiden Seiten einer transversalen Mittelachse erstrecken, wobei diese transversale Mittelachse der Fläche (5c) entspricht, die geeignet ist, gefaltet zu werden.

8. - Sterile Verpackung (1) nach einem der Ansprüche 1 bis 7, **dadurch gekennzeichnet, dass** der erste Teil (5) aus mehreren übereinander liegenden Lagen aus flexiblem und luftdichtem Material hergestellt ist.

9. - Sterilisierungsverfahren unter Verwendung einer sterilen Verpackung (1) nach einem der Ansprüche 1 bis 8, **dadurch gekennzeichnet, dass** es die Schritte umfasst:
- Platzieren des mindestens einen Objekts (2), das zu sterilisieren ist, in der Tasche (10), die von dem mindestens einen zweiten Teil (6) gebildet wird;
- Sterilisieren dieser Einheit mittels der Sterilisierungsflüssigkeit;
- Falten des zweiten Abschnitts (5b) auf den ersten Abschnitt (5a), und
- Ausführen des Zusammenfügens des zweiten Abschnitts (5b) auf den ersten Abschnitt (5a) in den Seitenmontageflächen (5d) mit den Verbindungsmitteln (11).

10. - Verfahren nach Anspruch 9, **dadurch gekennzeichnet, dass** es die Schritte umfasst:
- Verwenden des dritten Teils (7);
- Falten dieses dritten Teils (7) um den ersten Teil (5), wenn er um den zweiten Teil (6) gefaltet ist;
- Erzeugen eines Vakuums zwischen dem dritten Teil (7) und dem ersten Teil (5), und
- Abdichten des dritten Teils (7), während das Vakuum zwischen dem dritten Teil (7) und dem ersten Teil (5) aufrechterhalten wird.

11. - Verfahren nach Anspruch 10, **dadurch gekennzeichnet, dass** es die Schritte umfasst:
- Verbinden der Einheit, die von dem ersten Teil (5) gebildet wird, wenn dieser um den zweiten Teil (6) gefaltet ist, mit dem dritten Teil (7), insbesondere durch Verbinden der Seitenmontageflächen (5d) mit diesem dritten Teil (7).

## Revendications

1. - Emballage stérile (1) destiné à contenir au moins un objet (2) à stériliser par un fluide de stérilisation, comprenant :
- une première pièce (5), en matériau flexible et étanche à l'air, définissant une première partie (5a) et une deuxième partie (5b) attenante à la première partie (5a), ladite première partie (5a) étant séparée de la deuxième partie (5b) par une zone (5c) propre à être pliée ; et
- au moins une deuxième pièce (6), en matériau poreux au fluide de stérilisation, reliée à ladite première partie (5a) de ladite première pièce (5) ;
**caractérisé en ce que** :
- ladite au moins une deuxième pièce (6) forme une poche (10) de réception du ou des objets (2) à stériliser, ladite deuxième pièce (6) étant dimensionnée de telle sorte que ses bords soient en retrait des bords de ladite première partie (5a) de manière à définir sur ladite première partie (5a) des zones latérales d'assemblage (5d), ladite deuxième partie (5b) étant repliable sur ladite poche (10) et lesdites zones latérales d'assemblage (5d) et étant dimensionnée de manière à recouvrir l'ensemble de cette poche (10) et desdites zones latérales d'assemblage (5d), et étant reliée de manière étanche auxdites zones latérales d'assemblage (5d) de ladite première partie (5a), isolant de ce fait la poche (10) par rapport à l'extérieur, et
- l'emballage (1) inclut des moyens de liaison (11) pour réaliser une liaison étanche à l'air de ladite deuxième partie (5b) à ladite première partie (5a) au niveau desdites zones latérales d'assemblage (5d).

2. - Emballage stérile (1) selon la revendication 1, **caractérisé en ce qu'**il comprend une troisième pièce (7), faite en un matériau flexible et étanche à l'air, dimensionnée pour pouvoir contenir ladite première pièce (5) lorsque celle-ci est repliée sur ladite deuxième pièce (6), cette troisième pièce (7) étant refermée sur ladite première pièce (5) lorsque celle-ci est repliée sur ladite deuxième pièce (6) et étant scellée de manière étanche à l'air avec un vide d'air crée entre ladite troisième pièce (7) et ladite première pièce (5).

3. - Emballage stérile (1) selon la revendication 1 ou la revendication 2, **caractérisé en ce que** la ou lesdites deuxièmes pièces (6) forment par elles-mêmes ladite poche (10).

4. - Emballage stérile (1) selon la revendication 1 ou la revendication 2, **caractérisé en ce qu'**au moins une deuxième pièce (6) est plane et reliée à ladite première pièce (5) au niveau du pourtour de celle-ci, de manière à former ladite poche (10) et l'ouverture de cette poche (10).

5. - Emballage stérile (1) selon l'une des revendications 1 à 4, **caractérisé en ce que** ladite première pièce (5) est en matériau thermosoudable et **en ce que** lesdits moyens de liaison étanche comprennent ce matériau lui-même, l'assemblage de ladite première partie (5a) et de ladite deuxième partie (5b) de cette première pièce (5) étant réalisé par thermosoudure.

6. - Emballage stérile (1) selon l'une des revendications 1 à 5, **caractérisé en ce que** ladite deuxième pièce (6) est en matériau thermosoudable et **en ce que** sa liaison à ladite première pièce (5) est réalisée par thermosoudure.

7. - Emballage stérile (1) selon l'une des revendications 1 à 6, **caractérisé en ce que** ladite première pièce (5) présente une forme rectangulaire et **en ce que** lesdites première et deuxième parties (5a, 5b) de cette première pièce (5) sont définies par les deux parties de ladite première pièce (5) s'étendant de part et d'autre d'un axe transversal médian, cet axe transversal médian correspondant à ladite zone (5c) propre à être pliée.

8. - Emballage stérile (1) selon l'une des revendications 1 à 7, **caractérisé en ce que** ladite première pièce (5) est formée de plusieurs couches superposées de matériau flexible et étanche à l'air.

9. - Procédé de stérilisation, utilisant un emballage stérile (1) selon l'une des revendications 1 à 8, **caractérisé en ce qu'**il comprend les étapes consistant à :
- placer un ou plusieurs objets (2) à stériliser dans la poche (10) formée par ladite au moins une deuxième pièce (6) ;
- stériliser l'ensemble au moyen du fluide de stérilisation ;
- replier ladite deuxième partie (5b) contre ladite première partie (5a), et
- réaliser l'assemblage de cette deuxième partie (5b) à cette première partie (5a) au niveau desdites zones latérales d'assemblage (5d) grâce auxdits moyens de connexion (11).

10. - Procédé selon la revendication 9, **caractérisé en ce qu'**il comprend les étapes consistant à :
- utiliser ladite troisième pièce (7) ;
- refermer cette troisième pièce (7) sur ladite première pièce (5) lorsque celle-ci est repliée sur ladite deuxième pièce (6) ;
- créer un vide d'air entre ladite troisième pièce (7) et ladite première pièce (5), et
- sceller ladite troisième pièce (7) de manière étanche en maintenant le vide d'air entre ladite troisième pièce (7) et ladite première pièce (5).

11. - Procédé selon la revendication 10, **caractérisé en ce qu'**il comprend l'étape consistant à :
- relier l'ensemble formé par ladite première pièce (5) lorsque celle-ci est repliée sur ladite deuxième pièce (6), à ladite troisième pièce (7), en particulier en connectant lesdites zones latérales d'assemblage (5d) à cette troisième pièce (7).
